# EUROPEAN PATENT SPECIFICATION

(11) **EP 3 525 853 B1**
(45) Date of publication and mention of the grant of the patent: **25.02.2026**
(21) Application number: 17771758.4
(22) Date of filing: 22.09.2017
(51) Int. Cl.: A61M 15/00, A61M 5/31, A61M 5/24

(54) **HOUSING ELEMENT FOR A MEDICAMENT DELIVERY DEVICE, AN AUXILIARY UNIT FOR ATTACHMENT TO THE HOUSING ELEMENT, AND A MEDICAMENT DELIVERY DEVICE COMPRISING THE HOUSING ELEMENT**
GEHÄUSEELEMENT FÜR EINE MEDIKAMENTENABGABEVORRICHTUNG, HILFSEINHEIT ZUR BEFESTIGUNG AM GEHÄUSEELEMENT UND MEDIKAMENTENABGABEVORRICHTUNG MIT DEM GEHÄUSEELEMENT
BOÎTIER POUR DISPOSITIF D'ADMINISTRATION D'UN MÉDICAMENT, DISPOSITIF AUXILIAIRE DE FIXATION AU BOÎTIER ET DISPOSITIF D'ADMINISTRATION D'UN MÉDICAMENT COMPRENANT LE BOÎTIER

(30) Priority: 14.10.2016 WO PCT/EP2016/193873
(43) Date of publication of application: 21.08.2019
(73) Proprietor: SHL Medical AG, 6302 Zug (CH)
(72) Inventor: KELLER, Christian, Taipei 111 (TW); CIRILLO, William, Dublin 2 D02W D82 (IE)
(86) International application number: PCT/EP2017/074075
(87) International publication number: WO 2018/069022

(56) References cited:
- WO-A1-2009/032400
- WO-A1-2010/037828
- WO-A1-2013/120776
- WO-A1-2014/161952
- US-A- 3 982 716
- US-A1- 2005 192 557
- US-A1- 2012 053 527

## Description

### TECHNICAL FIELD

The present invention relates to a housing element for attaching an auxiliary unit to a medicament delivery device. More in particular, the housing element comprises an integrated mount for the auxiliary unit.

### BACKGROUND

To maintain optimum conditions, a patient suffering from a chronic medical condition is required to conform to a prescribed administration schedule of a medication, adhere to a prescribed dosage, avoid extra administrations, avoid missed administrations, and adhere to various recommended health and safety best practices.

The long-term health of the patient with a chronic medical condition depends on the day-to-day management of the condition. Mismanagement of the condition can result in significant morbidity and mortality and carry an increased risk of developing complications. Focused approach to management of a chronic medical condition is essential for the patient, in order to reduce the occurrence of these complications.

For example, diabetes occurs when the body does not produce enough insulin resulting for many diabetics in a requirement of a periodic insulin injection to control glucose levels in the body. One of the dangers associated with controlling glucose levels with insulin is insulin overdose. Symptoms of an insulin overdose reflect low blood sugar levels (hypoglycemia) and can include headache, irregular heartbeat, increased heart rate or pulse, sweating, tremor, nausea, increased hunger, and anxiety.

An insulin dependent diabetic needs to keep accurate track of the type and amount of insulin he is injecting. In order to help control the administration of insulin various supervisory devices have been developed. One prior art device, WO2010128493, comprises a particular sleeve being designed to rigidly lock onto to a prefabricated injection pen of a particular design, a universal header designed to rigidly lock onto the particular sleeve irrespective of the particular model of the prefabricated injection pen, an electronics assembly housed by the universal header, a sensor to detect an injection automatically, the sensor being communicatively coupled to the electronics assembly, a display to display injection data, and a button to allow manipulation and display of the injection data, including resetting the time.

This device suffers from a number of drawbacks. The sleeve is an extra component that the user has to cope with when administrating insulin, making drug administration more complicated. It may further have a less than perfect fit with the delivery device, which may cause it to detach and fall off. It is also prone to being dislocated, which may result in reduced quality of measurements and monitoring of the administration process.

WO 2010/037828 A1 discloses a medicament assembly with a monitoring device. US 2012/053527 A1 discloses a medicament injection supervisor device. WO 2013/120776 A1 discloses a supplemental device for attachment to an injection device. US 2005/192557 A1 discloses an integrated delivery device for continuous glucose sensor. WO 2014/161952 discloses a dose logging device for a drug delivery device. WO 2009/032400 A1 discloses an infusion pump system formed by a pump device and a controller device. US 3982716 A discloses a holder for temporarily supporting a bottle containing hypodermically injected medication.

### SUMMARY

An object of the present invention is to provide an improved device to remedy the drawbacks of prior art devices.

The invention is defined in the accompanying claims.

In the present application, when the term "distal" is used, this refers to the direction pointing away from the dose delivery site. When the term "distal part/end" is used, this refers to the part/end of the delivery device, or the parts/ends of the members thereof, which under use of the medicament delivery device is/are located furthest away from the dose delivery site. Correspondingly, when the term "proximal" is used, this refers to the direction pointing to the dose delivery site. When the term "proximal part/end" is used, this refers to the part/end of the delivery device, or the parts/ends of the members thereof, which under use of the medicament delivery device is/are located closest to the dose delivery site.

Further, the term "longitudinal", with or without "axis", refers to a direction or an axis through the device or components thereof in the direction of the longest extension of the device or the component.

The term "lateral", with or without "axis", refers to a direction or an axis through the device or components thereof in the direction of the broadest extension of the device or the component. "Lateral" may also refer to a position to the side of a "longitudinally" elongated body.

In a similar manner, the terms "radial" or "transversal", with or without "axis", refers to a direction or an axis through the device or components thereof in a direction generally perpendicular to the longitudinal direction, e.g. "radially outward" would refer to a direction pointing away from the longitudinal axis.

Also, if nothing else is stated, in the following description wherein the mechanical structure of the device and the mechanical interconnection of its components is described, the device is in an initial non-activated or non-operated state.

As stated above, the aim of the present invention is to provide a simple and robust orientation indicator that is also reliable and easy to use.

Optionally, the housing element is integral with an outer shell of a medicament delivery device.

The housing element is to be regarded as an integral part of the medicament delivery device. It may be a separate component before assembly, but during assembly it becomes an irreplaceable part of the delivery device. As such, a user may not dislocate it or remove it. An added advantage is that an auxiliary device which is attached to the mount is more robustly and accurately positioned in relation to the delivery device.

Optionally, the mount is positioned on a lateral side surface of the medicament delivery device.

Optionally, the mount is positioned on a distal end surface of the medicament delivery device.

Depending on the intended use of the auxiliary device, the position of the mount, in relation to the medicament delivery device, is chosen to achieve the best effect. For instance, a lateral position may be the most convenient for monitoring the movement of a plunger rod of the medicament delivery device, e.g. by optically monitoring the plunger rod through a window in the casing of the delivery device. On the other hand, in some instances it may be preferable to mount the auxiliary unit on the distal end, e.g. on a dose setting member.

Optionally, the mount is configured to releasably attach to an auxiliary unit by positive locking.

Optionally, the mount is configured to releasably attach to an auxiliary unit by friction-fit.

Optionally, the mount is configured to releasably attach to an auxiliary unit by magnetism.

Optionally, the mount is configured to releasably attach to an auxiliary unit by non-permanent adhesive.

The attachment between the mount and the auxiliary unit may be selected from a range of available methods. It is important, however, that the auxiliary device is securely fastened in a predetermined position, while still being removable from the mount.

Optionally, the mount is shaped to only allow attachment to a correspondingly shaped auxiliary unit.

Such a keyed attachment prevents a user from accidentally attaching an incorrect auxiliary unit to a medicament delivery device that he intends to use.

One embodiment is further characterized by an auxiliary unit for releasable attachment to the mount of said housing element, wherein the auxiliary unit is an electronics unit.

Optionally, the auxiliary unit is adapted to determine and to provide information about an operational state and/or usage of a medicament delivery device in which the housing element is comprised.

Optionally, the auxiliary unit is adapted to communicate measurements and information to another unit.

The electronics unit may comprise a processor capable of processing data program code for performing different tasks. The electronics unit may also comprise memory elements, in which retrieved data may be stored. The electronics unit may further comprise a power supply such as button cells, photovoltaic panels, etc. Communication elements of the electronics unit may communicate with another unit, such as a user or a smartphone, etc. The communication elements may comprise display elements that visual communication, e.g. by text on a display. In addition to, or instead, the user communication elements may comprise audio elements that can communicate audibly, via loudspeakers of the electronics unit. The communication elements may further comprise circuits for near range communication technology such as RFID, NFC or the like, as well as Bluetooth, Ant, ZigBee, just to mention a few.

Optionally, the auxiliary unit is correspondingly shaped with regard to the mount.

The corresponding shapes only allow a correct combination of a medicament delivery device with a predetermined auxiliary unit.

One embodiment is further characterized by a medicament delivery device comprising said housing element.

Optionally, the medicament delivery device is a disposable medicament delivery device.

Optionally, the medicament delivery device is a pen injector.

Optionally, the medicament delivery device is an inhalation device.

A further advantage of the present invention is that it may be adapted for disposable medicament delivery devices, where the user removes the auxiliary device from the mount and disposes of the medicament delivery device, for instance by putting it in a sharps container, when the delivery device is expended. The auxiliary unit may then be reused and attached to a new disposable device. Pen injectors represent a type of delivery device that is often disposable. They are often used for administration of insulin.

These and other features and advantages of the present invention will become apparent from the following detailed example of an embodiment and the accompanying drawings.

### BRIEF DESCRIPTION OF THE DRAWINGS

In the following detailed description, reference will be made to the accompanying drawing of which
- Fig. 1: shows a perspective view of a medicament delivery device comprising a housing element according to the invention,
- Fig. 2A-D: show perspective views of a housing element comprising a mount according to the invention,
- Fig. 3A-C: show perspective views of an auxiliary unit arranged to be attached to the mounts of Fig. 2A-D, and
- Fig. 4A-B: show perspective views of a housing element comprising a mount according to an alternative embodiment invention

### DETAILED DESCRIPTION

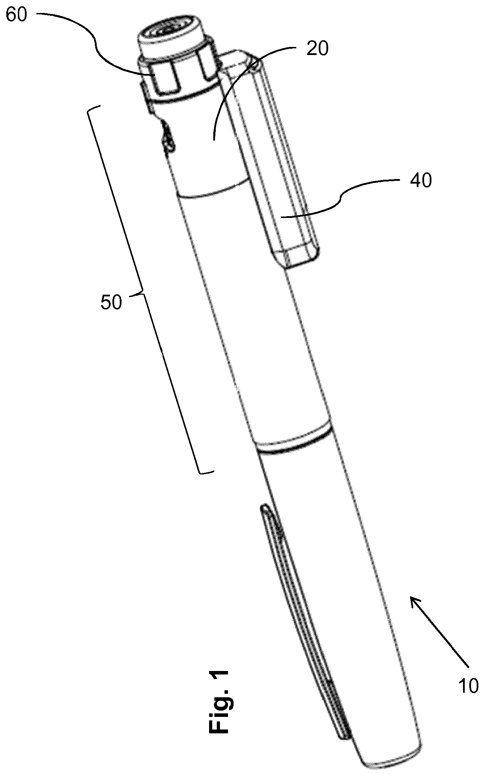

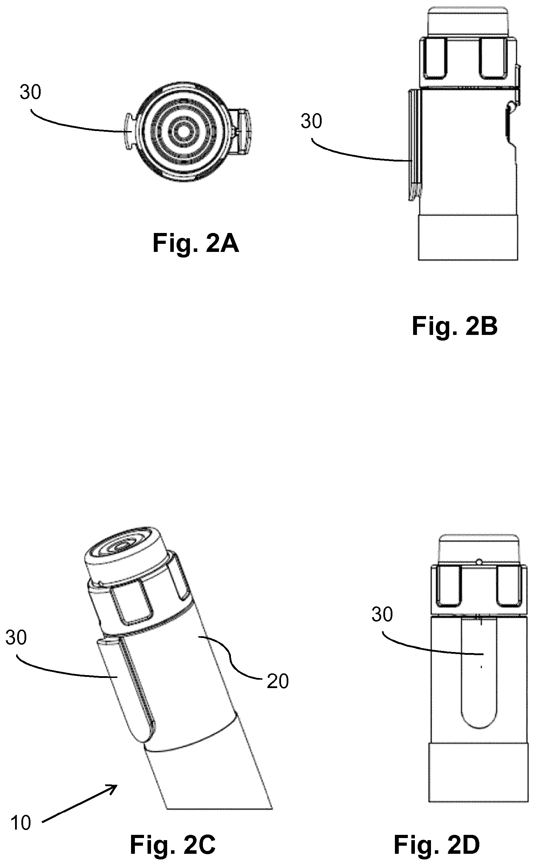

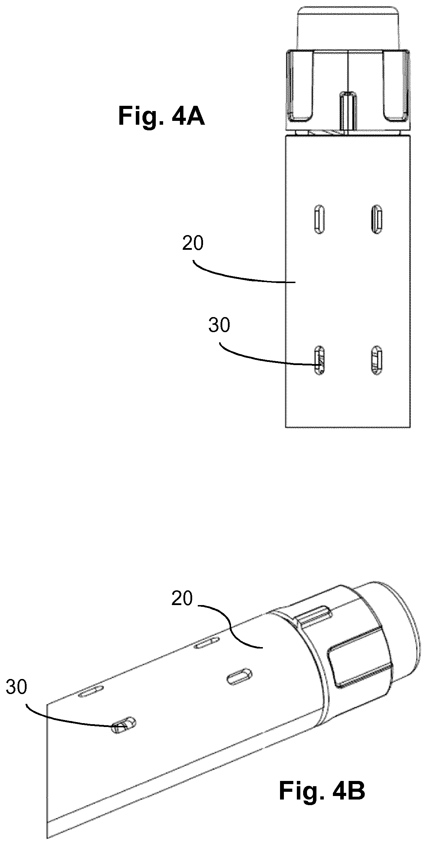

Fig. 1 shows a perspective view of a medicament delivery device 10 comprising a housing element 20 according to an embodiment of the present invention. The housing element comprises a mount 30, Figs 2 and 4, for releasably attaching an auxiliary unit 40. The mount 30 is integral with the housing element 20. As such, the mount is fixedly attached to, or unitary with, the housing element 20. The housing element 20 may in turn be an individual component of a medicament delivery device 10 before assembly, but during assembly it becomes an irreplaceable or non-removable part of the medicament delivery device 10. In one embodiment, the housing element 20 may be a unitary or integral part of a larger housing 50, Fig. 2, e.g. an outer casing, of the medicament delivery device 10. Integration of the mount 30 with a fixed part of the medicament delivery device 10 ensures that the auxiliary unit 40 will be robustly and accurately positioned in relation to the delivery device.

The housing element 20 of Fig. 1 is shown as a tubular element, but it may essentially have any form as long as it is at least configured to be fixed, or non-removably attached, to a medicament delivery device.

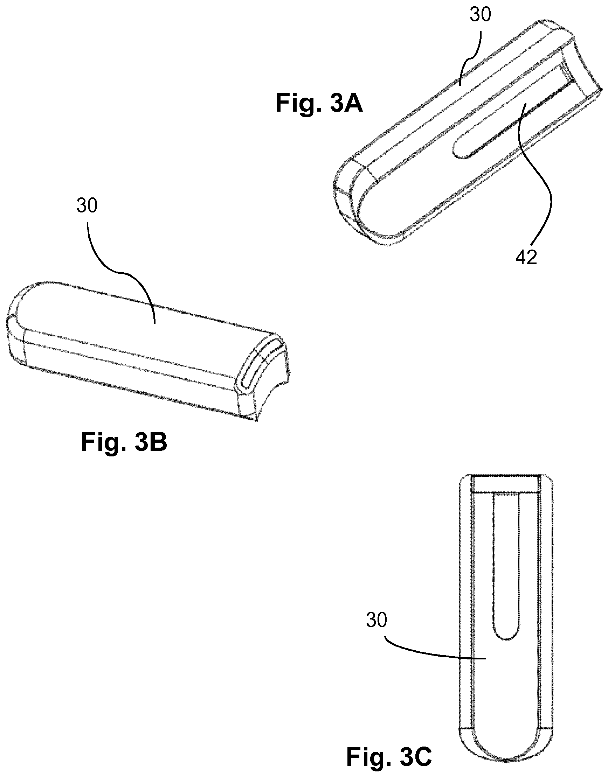

The mount 30 of Figs 2 and 3 is positioned on lateral side surface of the medicament delivery device 10. The position of the mount is chosen to improve the measuring and sensing characteristics of the auxiliary unit 40 that is intended to be attached to the mount 30.

For instance, it may be advantageous to position the mount 30 and the auxiliary unit 40 on the lateral side surface of the medicament delivery device 10 in order detect relative axial movements of housing parts of the delivery device10, e.g. movement of a dose setting member 60 in relation to the housing 50. In some cases, however, a position on a distal end surface of the medicament delivery device 10 may be more useful. The position of the mount 30 and the auxiliary unit 40 must also be positioned such that they support user interaction with the auxiliary unit 40 without hindering normal use of the delivery device 10.

There are many different ways in which the auxiliary unit 40 may be releasably attached to the mount 30. Examples include positive locking, friction-fit, magnetism, or non-permanent adhesive.

The mount 30, as exemplified by Figs 2 and 3, may be achieve attachment to the auxiliary unit 40 by positive locking of members such as snaps, latches, hooks, gripping members, or the like, to allow quick and reliable assembly of the mount 30 and the auxiliary unit 40. As one example of a positive locking shown in Figs. 2, the mount 30 has a dovetail shape as seen in a cross-sectional view. The auxiliary unit 40 is now arranged with a groove 42 that has a complementary shape so that the auxiliary unit 40 may be pushed onto the mount 30 and be held there by the complementary shapes. Positive locking is furthermore easily provided by molding the locking members as integral parts of the mount 30 and of the auxiliary unit 40 during manufacturing.

Likewise, friction-fit is easily achieved in the same manner. Friction-fit is advantageous in that the mount and the auxiliary unit may be detached without unlocking any locking members.

Attachment by magnets or non-permanent adhesive is preferably combined with a shaped form-fit such that the relative positions of the mount 30 and the auxiliary unit 40 is uniquely determined by their complimentary shapes as they dock with each other.

Fig. 2A-D show a mount 30 protruding from the housing element 20, but the mount may alternatively be formed as slots, holes or recesses in the housing element 20, as shown in Figs 4A-B. In this case, the auxiliary unit comprises corresponding protrusions that may be inserted in the slots, holes or recesses, in order to removably lock the auxiliary unit 40 to the mount 30.

Regardless of the type of attachment, the mount 30 and the auxiliary unit 40 may be keyed to each other by corresponding shapes or positions of the respective attachment devices such that a particular auxiliary unit 40 may only be attached to a certain mount 30 of a housing element 20, comprised in a predetermined medicament delivery device 10. Such a keyed attachment only allows the correct auxiliary unit 40 to be attached to the intended medicament delivery device 10.

The various types of attachment devices, e.g. snaps, latches, hooks, gripping members, slots, grooves, holes, recesses, etc., are not shown in the drawings since they are obvious variations that are readily available to a skilled person.

The auxiliary unit 40 shown in Fig. 1 may be an electronics unit, i.e. a module comprising electronics and a power supply such as button cells, photovoltaic panels, etc. The electronics unit may comprise a processor capable of processing data program code for performing different tasks such as instructing and informing the user on the handling of the medicament delivery device 10 and on the progress or status of the drug administration. The electronics unit may also comprise memory elements, in which retrieved data may be stored. The electronics unit may also transmit and/or receive data to and/or from other units, such as smartphones, computers, the user and/or a physician, via the communication elements, which may comprise circuits for near range communication technology, exemplified by RFID, NFC or the like, as well as Bluetooth, Ant and/or ZigBee. The electronics unit may also comprise display elements for visual communication, e.g. by text on a display. The electronics unit may furthermore comprise audio elements that can communicate audibly, via loudspeakers of the electronics unit.

In use, a medicament delivery device 10 is unpacked by the user. The auxiliary unit 40 is thereafter removably attached to the mount 30, which is integrated with the housing element 20 of the medicament delivery device 10. In the example of Figs 2A-D and 3A-C, the auxiliary unit 40 is axially slid onto the mount 30. Snaps or latches may lock it in place. Drug administration may then be carried out according to the handling instructions of the delivery device in question. If the auxiliary unit 40 is an electronics unit, the electronics unit may, for instance, be activated when the user operates the medicament delivery device 10 to set a dose. The electronics unit may then carry out its pre-defined tasks, such as data collection and/or transmission of information, e.g. until the dose has been fully delivered, whereupon the electronics unit may be de-activated.

The user may then remove the medicament delivery device 10 from the administration site, detach the auxiliary unit 40, and discard the medicament delivery device 10, for instance in a sharps container, if the delivery device is a needle-based injection device.

The auxiliary unit 40 is kept by the user for later use with another medicament delivery device 10. In this way, the auxiliary unit is used for a number of delivery devices, possibly for the whole treatment of an affliction. In the case where the auxiliary unit 40 is an electronics unit, the data collected may be analysed and used to improve the therapy of a patient/user.

It should be understood that the housing element described above and shown in the drawings is to be regarded only as a non-limiting example and that it may be modified in many way within the scope of the patent claims.

## Claims

1. A medicament delivery device 10 comprising a housing element 20, which housing element 20 comprises a mount 30 for releasably attaching an auxiliary unit 40, and wherein the mount 30 is integral with the housing element 20;
wherein the housing element 20 is integral with an outer shell 50 of a medicament delivery device 10;
wherein the medicament delivery device 10 is a disposable medicament delivery device,
wherein the medicament delivery device 10 is a pen injector, wherein the housing element is an integral part of the medicament delivery device,
wherein the housing element 20 is tubular,
**characterised in that** the mount 30 is configured to releasably attach to an auxiliary unit 40 by magnetism; and
wherein the mount 30 is formed as slots, holes or recesses in the housing element 20.

2. A medicament delivery device 10 according to claim 1, wherein the mount 30 is positioned on a lateral side surface of the medicament delivery device 10.

3. A medicament delivery device 10 according to claim 1, wherein the mount 30 is positioned on a distal end surface of the medicament delivery device 10.

4. An auxiliary unit 40 for releasable attachment to the mount 30 of the housing element 20 of the medicament delivery device 10 according to any of the claims 1-3, wherein the auxiliary unit 40 is an electronics unit.

5. An auxiliary unit according to claim 4, wherein the auxiliary unit 40 is adapted to determine and to provide information about an operational state and/or usage of the medicament delivery device 10 in which the housing element 20 is comprised.

6. An auxiliary unit according to claim 5, wherein the auxiliary unit 40 is adapted to communicate measurements and information to another unit.

7. An auxiliary unit 40 for releasable attachment to the mount 30 of the housing element 20 according to claim 4, wherein the auxiliary unit is correspondingly shaped with regard to the mount 30.

## Patentansprüche

1. Medikamentenabgabevorrichtung 10, umfassend ein Gehäuseelement 20,
wobei das Gehäuseelement 20 eine Halterung 30 zum lösbaren Befestigen einer Hilfseinheit 40 umfasst, und wobei die Halterung 30 integral mit dem Gehäuseelement 20 ist;
wobei das Gehäuseelement 20 integral mit einer äußeren Hülle 50 einer Medikamentenabgabevorrichtung 10 ist;
wobei die Medikamentenabgabevorrichtung 10 eine Einweg-Medikamentenabgabevorrichtung ist,
wobei die Medikamentenabgabevorrichtung 10 ein Stift-Injektor ist,
wobei das Gehäuseelement ein integraler Bestandteil der Medikamentenabgabevorrichtung ist,
wobei das Gehäuseelement 20 rohrförmig ist,
**dadurch gekennzeichnet, dass** die Halterung 30 konfiguriert ist, um lösbar an einer Hilfseinheit 40 durch Magnetismus befestigt zu werden; und
wobei die Halterung 30 als Schlitze, Löcher oder Vertiefungen in dem Gehäuseelement 20 ausgebildet ist.

2. Medikamentenabgabevorrichtung 10 nach Anspruch 1, wobei die Halterung 30 auf einer lateralen Seitenoberfläche der Medikamentenabgabevorrichtung 10 positioniert ist.

3. Medikamentenabgabevorrichtung 10 nach Anspruch 1, wobei die Halterung 30 auf einer distalen Endoberfläche der Medikamentenabgabevorrichtung 10 positioniert ist.

4. Hilfseinheit 40 zum lösbaren Befestigen an der Halterung 30 des Gehäuseelements 20 der Medikamentenabgabevorrichtung 10 nach einem der Ansprüche 1 bis 3, wobei die Hilfseinheit 40 eine Elektronikeinheit ist.

5. Hilfseinheit nach Anspruch 4, wobei die Hilfseinheit 40 angepasst ist, um Informationen über einen Betriebszustand und/oder eine Verwendung der Medikamentenabgabevorrichtung 10, in der das Gehäuseelement 20 enthalten ist, zu bestimmen und bereitzustellen.

6. Hilfseinheit nach Anspruch 5, wobei die Hilfseinheit 40 angepasst ist, um Messungen und Informationen an eine andere Einheit zu übermitteln.

7. Hilfseinheit 40 zum lösbaren Befestigen an der Halterung 30 des Gehäuseelements 20 nach Anspruch 4, wobei die Hilfseinheit entsprechend bezüglich der Halterung 30 geformt ist.

## Revendications

1. Dispositif de délivrance de médicament 10 comprenant un élément de logement 20,
cet élément de logement 20 comprenant une monture 30 permettant d'attacher de façon libérable une unité auxiliaire 40, et dans lequel la monture 30 est solidaire de l'élément de logement 20 ;
dans lequel l'élément de logement 20 est solidaire d'une enveloppe externe 50 d'un dispositif de délivrance de médicament 10 ;
dans lequel le dispositif de délivrance de médicament 10 est un dispositif jetable de délivrance de médicament,
dans lequel le dispositif de délivrance de médicament 10 est un stylo injecteur,
dans lequel l'élément de logement fait partie intégrante du dispositif de délivrance de médicament,
dans lequel l'élément de logement 20 est tubulaire,
**caractérisé en ce que** la monture 30 est conçue pour s'attacher de manière libérable à une unité auxiliaire 40 par magnétisme ; et
dans lequel la monture 30 est formée en tant que fentes, trous ou renfoncements dans l'élément de logement 20.

2. Dispositif de délivrance de médicament 10 selon la revendication 1, dans lequel la monture 30 est positionnée sur une surface latérale du dispositif de délivrance de médicament 10.

3. Dispositif de délivrance de médicament 10 selon la revendication 1, dans lequel la monture 30 est positionnée sur une surface d'extrémité distale du dispositif de délivrance de médicament 10.

4. Unité auxiliaire 40 pour attachement libérable à la monture 30 de l'élément de logement 20 du dispositif de délivrance de médicament 10 selon l'une quelconque des revendications 1 à 3, dans laquelle l'unité auxiliaire 40 est une unité électronique.

5. Unité auxiliaire selon la revendication 4, dans laquelle l'unité auxiliaire 40 est conçue pour déterminer et pour fournir des informations concernant un état opérationnel et/ou une utilisation du dispositif de délivrance de médicament 10 dans lequel l'élément de logement 20 est compris.

6. Unité auxiliaire selon la revendication 5, dans laquelle l'unité auxiliaire 40 est conçue pour communiquer des mesures et des informations à une autre unité.

7. Unité auxiliaire 40 pour attachement libérable à la monture 30 de l'élément de logement 20 selon la revendication 4, dans laquelle l'unité auxiliaire est mise en forme de manière correspondante par rapport à la monture 30.
